# EUROPEAN PATENT SPECIFICATION

(11) **EP 1 062 865 B1**
(45) Date of publication and mention of the grant of the patent: **21.11.2001**
(21) Application number: 99112162.5
(22) Date of filing: 24.06.1999
(51) Int. Cl.: A01H 3/00

(54) **Method for propagating campanula**
Verfahren zum Vermehren von Campanula
Méthode pour la propagation de campanula

(43) Date of publication of application: 27.12.2000
(73) Proprietor: Madsen, Kristian, 5270 Odense (DK)
(72) Inventor: Madsen, Kristian, 5270 Odense (DK)
(74) Representative: Roerboel, Leif

(56) References cited:
- US-A- 3 868 054
- US-A- 5 401 281
- DATABASE CAB [Online] CAB INTERNATIONAL, WALLINGFORD, OXON, GB AN - 80:12836, 1979 HOLCOMB, E.: "Growing Reiger begonias from terminal cuttings" XP002124862
- PATENT ABSTRACTS OF JAPAN vol. 1998, no. 03, 27 February 1998 (1998-02-27) & JP 09 294462 A (NETSUTAIRIN SAISEI GIJUTSU KENKYU KUMIAI), 18 November 1997 (1997-11-18)

## Description

The present invention relates to a method of industrial propagation of Campanula by using cuttings from another Campanula plant, said method being of the kind set forth in the preamble of claim 1.

### TECHNICAL FIELD

Up to the present moment, methods of the kind referred to above have been carried out by planting a single cutting in a container filled with a growing substrate. In a first stage, the container with the newly planted cutting was then placed in a greenhouse until the plant was strong enough to be placed outside to develop further. The plants were allowed to grow until they were nearly full mature, i.e. at a size suitable for sale, upon which they were placed back in the greenhouse for a final growing stage before sales.

One of the problems encountered with the prior art method is that the total growing period is very long since the plants develop slowly outdoors. Thus, it was difficult to react quickly to market changes. Further, being placed outside the greenhouse the plants pick up fungi that are nearly impossible to completely dispose of.

### DISCLOSURE OF THE INVENTION

On the background of the above, it is the object of the invention to provide a method of the kind referred to initially, with properties much more favourable than corresponding prior art methods. This object is achieved by proceeding in the manner set forth in the characterising clause of claim 1. By proceeding in this manner, at least two cuttings are planted spaced over the surface of the substrate in the container reducing the production period of the culture. This renders a pure greenhouse production economical. Moreover, the plants have more stems to stand on making them more stable. By avoiding the outdoor period the fungi problem is avoided.

### BRIEF DESCRIPTION OF THE DRAWINGS

In the following detailed portion of the present description, the invention will be explained in more detail with reference to exemplary embodiments according to the invention as shown in the drawings, in which
Figure 1 shows a sales pot with the plants arranged according to the prior art method,
Figure 2 shows a multiple-cell tray, and
Figure 3 shows a sales pot with the plants arranged according to the present invention.

### DETAILED DESCRIPTION OF THE INVENTION

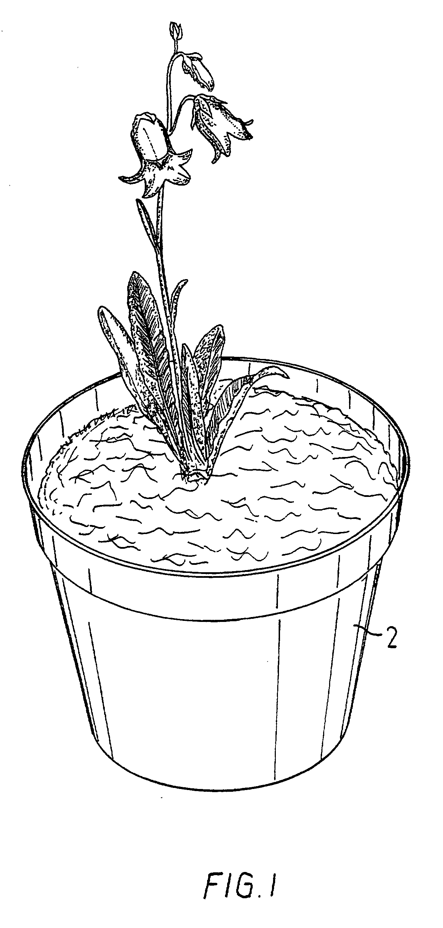

Figure 1 shows a developed plant in a sales pot 2 when it is grown by placing a single cutting in a pot according to the prior art method. The single cutting 1 has been planted in the sales pot and after a certain period in the greenhouse, i.e. relatively short to be acceptable from an economic point of view, the plant has, as can be seen from Figure 1, not developed to a stage where it is ready for sales.

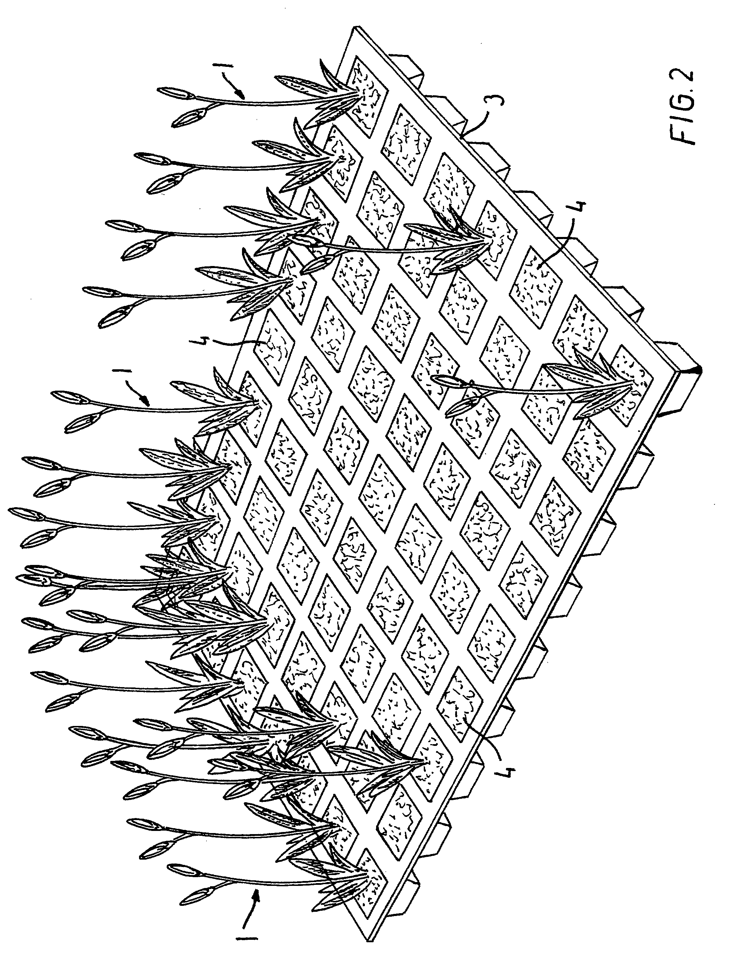

In the method according to the invention, the cuttings 1 may undergo a rooting and growing phase in a multiple-cell tray in the greenhouse as shown in Figure 2. The multiple-cell tray 3 comprises a plurality of relatively small cells 4. The space that is available for the cuttings 1 to strike root and grow in a compartment 4 is therefore limited. The tray has been designed according to certain standards to allow automatic re-potting equipment (not shown) to carry out the re-potting operation.

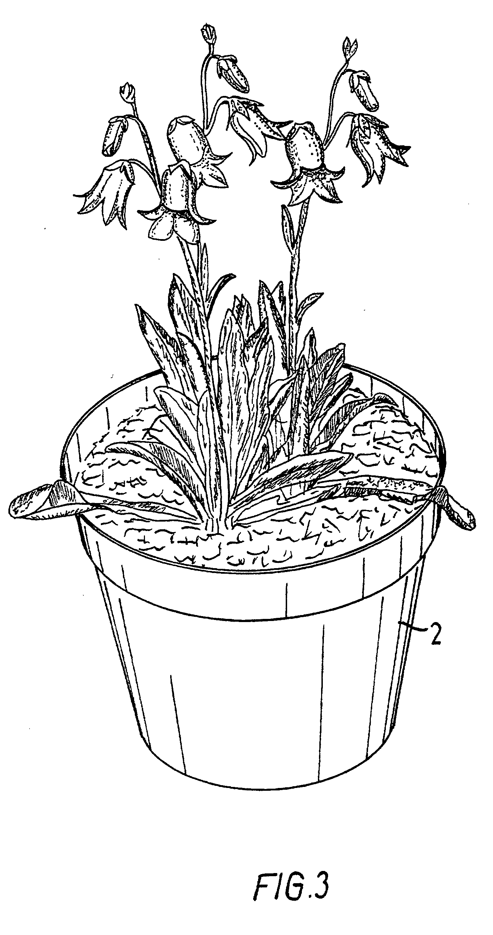

After the initial phase in the multiple-cell tray 3, the germinated cuttings 1 are placed over into a sales pot 2. The germinated cuttings 1 are potted over into a sales pot 2 in sets of at least two plants. The two or more plants are then allowed to grow to a mature plant in the greenhouse (not shown). As can be seen from Figure 3, the group of plants will reach a size and appearance which is acceptable for sales after a relatively short period in the greenhouse.

Alternatively, the cuttings 1 can be planted in sets of two or more directly in the growing substrate of the sales pot 2, without using a multiple-cell tray 3.

Generally it has proven that sets with two to six cuttings 1 or germinated cuttings 1 planted as a set in the sales pot give the best result.

For Campanula of the type "Blue Wonder", the set comprises two to three cuttings 1 or germinated cuttings 1 in order to achieve the best result. For Campanula of the type "Elizabeth Oliver", the set comprises two to five cuttings 1 or germinated cuttings 1 for the best result.

According to an embodiment of the invention, the plants are exposed to a cooling period in the greenhouse, preferably every night, during their development to a mature plant for those types of Campanula that require such treatment. Typically Campanula which originate from a natural habitat with cold nights require such a treatment for optimum development.

The sales pots are filled with the growing substrate that best suits the circumstances and the type of Campanula. Suitable growing substrates are e.g. peat, soil, compost, crushed mineral wool, perlite, vermiculite or bark.

The cuttings used for the propagation can be provided by taking them from a special mother plant or by taking them from sales plants.

The present method of propagating Campanula is especially useful in combination with semi-automatic or automatic equipment (not shown) to handle the different steps defined by the method. This equipment is used e.g. to fill the growing substrate into the tray 3 or pot 2 and to drill holes in the growing substrate that receive the plants or cuttings. Such equipment is in itself well-known in the art and will not be described in detail here.

### LIST OF REFERENCE NUMERALS

- 1: cutting or germinated cutting
- 2: container or sales Dot
- 3: multiple-cell tray
- 4: cell
- 6: substrate

## Claims

1. Method of propagating Campanula comprising the steps of:
- providing a greenhouse,
- providing cuttings (1) from a Campanula plant,
- providing a container (2), e.g. a pot, with a growing substrate (6),
- planting the cuttings (1) in the growing substrate in said container (2) in sets of at least two cuttings (1) such that the cuttings (1) are spaced apart over the surface area of said growing substrate (6),
- placing the containers (2) with the newly planted cuttings (1) in the greenhouse, and
- allowing the sets of cuttings (1) to germinate and grow to a mature plant in the greenhouse.

2. Method of propagating Campanula comprising the steps of:
- providing a greenhouse,
- providing cuttings (1) from a Campanula plant,
- providing a tray (3) with a plurality of cells (4) filled with a growing substrate,
- planting one cutting (1) in each of the cells (4),
- placing the trays (3) with the newly planted cuttings (1) in the greenhouse, and
- allowing the cuttings (1) a germination and initial growing stage in the cells (4),
- providing a container (2), e.g. a pot, with a growing substrate (6),
- planting the germinated cuttings (1) in the growing substrate in said container (2) in sets of at least two germinated cuttings (1) such that the germinated cuttings (1) are spaced apart over the surface area of said growing substrate (6),
- allowing the sets of germinated cuttings (1) to grow to a mature plant in the greenhouse.

3. Method according to claim 1 or 2, **characterised in that** a set comprises two to six cuttings (1) or germinated cuttings (1).

4. Method according to claim 3, **characterised in that** for Campanula of the type "Blue Wonder" the set comprises two to three cuttings or germinated cuttings (1).

5. Method according to claim 3, **characterised in that** for Campanula of the type "Elizabeth Oliver" the set comprises two to five cuttings or germinated cuttings (1).

6. Method according to any of the claims 1-5, **characterised in that** cuttings (1) are exposed to a cooling period in the greenhouse, preferably every night, during their development to a mature plant.

7. Method according to any of the claims 1-6, **characterised in that** the container (2) is a sales pot.

8. Method according to claims 1-7, **characterised in that** the growing substrate (6) is peat, compost, soil, crushed mineral wool, perlite, vermiculite, bark or mixtures thereof.

9. Method according to any of the claims 1-8, **characterised in that** one or more of the steps of the method, in particular the planting step, is carried out by automatic equipment.

## Patentansprüche

1. Verfahren zur Vermehrung von Campanula, umfassend die Schritte:
- ein Gewächshaus zur Verfügung zu stellen,
- Stecklinge (1) von einer Campanula-Pflanze bereitzustellen,
- ein Behältnis (2), zum Beispiel einen Topf, mit einem Wachstumssubstrat (6) bereitzustellen,
- die Stecklinge (1) in das Wachstumssubstrat im Behältnis (2) in Gruppen von mindestens zwei Stecklingen (1) zu pflanzen, so dass die Stecklinge (1) einzeln über die Oberfläche des Wachstumssubstrats (6) verteilt sind,
- die Behältnisse (2) mit den frisch gepflanzten Stecklingen (1) in das Gewächshaus zu stellen, und
- die Gruppen der Stecklinge (1) im Gewächshaus keimen und zu einer reifen Pflanze wachsen zu lassen.

2. Verfahren zur Vermehrung von Campanula, umfassend die Schritte:
- ein Gewächshaus zur Verfügung zu stellen,
- Stecklinge (1) von einer Campanula-Pflanze bereitzustellen,
- einen mit einem Wachstumssubstrat gefüllten Pflanztrog (3) mit einer Vielzahl von Fächern (4) bereitzustellen,
- einen Steckling (1) in jedes der Fächer (4) zu pflanzen,
- die Pflanztröge (3) mit den frisch gepflanzten Stecklingen (1) in das Gewächshaus zu stellen, und
- den Stecklingen (1) eine Keimung und anfängliche Wachstumsphase in den Fächern (4) zu ermöglichen,
- ein Behältnis (2), zum Beispiel einen Topf, mit einem Wachstumssubstrat (6) bereitzustellen,
- die gekeimten Stecklinge (1) in das Wachstumssubstrat im Behältnis (2) in Gruppen von mindestens zwei gekeimten Stecklingen (1) zu pflanzen, so dass die gekeimten Stecklinge (1) einzeln über die Oberfläche des Wachstumssubstrats (6) verteilt sind,
- die Gruppen der gekeimten Stecklinge (1) im Gewächshaus zu einer reifen Pflanze wachsen zu lassen.

3. Verfahren nach Anspruch 1 oder 2, **dadurch gekennzeichnet, dass** eine Gruppe zwei bis sechs Stecklinge (1) oder gekeimte Stecklinge (1) umfaßt.

4. Verfahren nach Anspruch 3, **dadurch gekennzeichnet, dass** für Campanula der Art "Blue Wonder" die Gruppe zwei bis drei Stecklinge oder gekeimte Stecklinge (1) umfaßt.

5. Verfahren nach Anspruch 3, **dadurch gekennzeichnet, dass** für Campanula der Art "Elizabeth Oliver" die Gruppe zwei bis fünf Stecklinge oder gekeimte Stecklinge (1) umfaßt.

6. Verfahren nach einem der Ansprüche 1 bis 5, **dadurch gekennzeichnet, dass** die Stecklinge (1) während ihrer Entwicklung zu einer reifen Pflanze im Gewächshaus einer Kühlperiode ausgesetzt werden, bevorzugt jede Nacht.

7. Verfahren nach einem der Ansprüche 1 bis 6, **dadurch gekennzeichnet, dass** das Behältnis (2) ein Verkaufstopf ist.

8. Verfahren nach einem der Ansprüche 1 bis 7, **dadurch gekennzeichnet, dass** das Wachstumssubstrat (6) Torf, Kompost, Erde, zerkleinerte Mineralwolle, Perlit, Vermiculit, Rinde oder Gemische davon ist.

9. Verfahren nach einem der Ansprüche 1 bis 8, **dadurch gekennzeichnet, dass** einer oder mehrere der Verfahrensschritte, insbesondere der Pflanzschritt, mit einer automatischen Ausrüstung ausgeführt werden.

## Revendications

1. Méthode pour propager la campanule comprenant les étapes consistant à :
- fournir une serre,
- fournir des boutures (1) d'une plante de campanule,
- fournir un récipient (2), par exemple un pot, avec un substrat de culture (6),
- planter des boutures (1) dans le substrat de culture dans ledit récipient (2) en séries d'au moins deux boutures (1) de telle façon que les boutures (1) soient séparées les unes des autres sur l'aire de surface dudit substrat de culture (6),
- disposer les récipients (2) avec les boutures (1) nouvellement plantées dans la serre, et
- permettre aux séries de boutures (1) de germer et de croître jusqu'à une plante adulte dans la serre.

2. Méthode pour propager la campanule comprenant les étapes consistant à :
- fournir une serre,
- fournir des boutures (1) d'une plante de campanule,
- fournir un plateau (3) avec une pluralité de cellules (4) remplies de substrat de culture,
- planter une bouture (1) dans chacune des cellules (4),
- disposer des plateaux (3) avec les boutures (1) nouvellement plantées dans la serre, et
- permettre aux boutures (1) de germer et de suivre le stade initial de croissance dans les cellules (4),
- fournir un récipient (2), par exemple un pot, avec un substrat de culture (6),
- planter les boutures (1) germées dans le substrat de culture dans ledit récipient (2) en série d'au moins deux boutures (1) germées de telle façon que les boutures (1) germées soient séparées les unes des autres sur l'aire de surface dudit substrat de culture (6),
- permettre aux séries de boutures (1) germées de croître jusqu'à une plante adulte dans la serre.

3. Méthode selon la revendication 1 ou 2, **caractérisée en ce qu'**une série comprend deux à six boutures (1) ou boutures (1) germées.

4. Méthode selon la revendication 3, **caractérisée en ce que** pour la campanule du type « Blue Wonder », la série comprend deux à trois boutures (1) ou boutures (1) germées.

5. Méthode selon la revendication 3, **caractérisée en ce que** pour la campanule du type « Elizabeth Oliver », la série comprend deux à cinq boutures (1) ou boutures (1) germées.

6. Méthode selon l'une quelconque des revendications 1-5, **caractérisé en ce que** les boutures (1) sont exposées à une période de refroidissement dans la serre, de préférence chaque nuit, au cours de leur développement en plante adulte.

7. Méthode selon l'une quelconque des revendications 1-6, **caractérisé en ce que** le récipient (2) est un pot du commerce.

8. Méthode selon l'une quelconque des revendications 1-7, **caractérisé en ce que** le substrat de culture (6) est une tourbe, compost, terre, laine minérale écrasée, perlite, vermiculite, écorce ou un mélange de ceux-ci.

9. Méthode selon l'une quelconque des revendications 1-8, **caractérisé en ce qu'**une étape ou plus de la méthode, en particulier l'étape de plantation, est mise en oeuvre par un équipement automatique.
